# EUROPEAN PATENT SPECIFICATION

(11) **EP 1 774 962 B1**
(45) Date of publication and mention of the grant of the patent: **19.03.2014**
(21) Application number: 05028228.4
(22) Date of filing: 22.12.2005
(51) Int. Cl.: A61K 9/127, A61K 48/00, C12N 15/88

(54) **Lipid carrier and method of preparing the same**
Lipidträger und ihre Herstellung
Vecteur lipidique et son procédé de préparation

(30) Priority: 14.10.2005 US 249326
(43) Date of publication of application: 18.04.2007
(73) Proprietor: INDUSTRIAL TECHNOLOGY RESEARCH INSTITUTE, Chutung, Hsinchu (TW)
(72) Inventor: Li, Shyh-Dar, Miaoli Conty 351 (TW); Wang, Ae-June, Hsinchu (TW)
(74) Representative: Ter Meer Steinmeister & Partner

(56) References cited:
- WO-A-2005/121348
- WO-A2-98/51278
- WO-A2-2004/089339
- GIRAO DA CRUZ M T ET AL: "Improving lipoplex-mediated gene transfer into C6 glioma cells and primary neurons" EXPERIMENTAL NEUROLOGY, ACADEMIC PRESS, NEW YORK, NY, US, vol. 187, no. 1, May 2004 (2004-05), pages 65-75, XP004620583 ISSN: 0014-4886
- JEFFS LLOYD B ET AL: "A scalable, extrusion-free method for efficient liposomal encapsulation of plasmid DNA" PHARMACEUTICAL RESEARCH (DORDRECHT), vol. 22, no. 3, March 2005 (2005-03), pages 362-372, XP002422317 ISSN: 0724-8741

## Description

### BACKGROUND

The invention relates to a lipid carrier, and more specifically to a pH-sensitive and serum-resistant lipid carrier and a method of preparing the same.

Gene therapies have gained increasing popularity in medical treatment, especially for hereditary or acquired diseases, such as primary immune deficiencies and cancers. Gene therapy has entered in vivo experiment and human clinical trial from vitro experiment in the past fifteen years. Although the clinical trials of gene therapy are still in phase I or II, gene therapy appears promising in the future. Generally speaking, carriers delivering gene drugs are divided into viral and non-viral two types, wherein viral carriers are used in 85% of clinical trials currently.

Nevertheless, in September 1999, a teenager with Ornithine Transcarbamylase Deficiency (OTC) died during treatment with gene therapy at Pennsylvania Medicine Center. In February of the following year, the FDA stopped all clinical trials of gene therapy in U.S.A. In addition, a child with X-linked Severe Combined Immune Deficiency (X-SCID) developed leukemia after treatment with gene therapy in September 2002 in France. Unfortunately, a second leukemia case occurred in 2003 in the same clinical trial. Main virulence factors were determined to be immunogenecity and toxicity from viral carriers. Thus, development of a non-viral gene trnasfection system became a critical point in gene therapy. Currently, non-viral carriers include lipid and polymer based carriers, with lipid carriers discussed herein.

Cationic lipid carriers, most commonly used, present the highest transfection efficiency among non-viral carriers. There are several in vitro reagents available presently, such as Lipofectin and Lipofectamine from Life Technologies, applicable in gene and protein researches. Besides high transfection efficiency, cationic lipid carriers also provide the advantage of endosomolysis activity and high biocompatibility. Nevertheless, the cationic lipid and DNA complex generally exhibit a diameter exceeding 500nm, and also are unstable in serum. Thus, the complex is mainly used in vitro study or for local injection. The DC-liposome provided by Dr. Leaf Huang was the first lipid carrier and enters clinical trial for the treatment of breast cancer by local injection. Currently, the DC-liposome is in phase II clinical trial.

In order to reduce the large particle size of cationic lipid carrier and DNA complex, Dr. Leaf Huang provides a novel gene transfection system called LPD with addition of cationic peptides, such as protamine or polylysine, to reduce the particle size and also to increase transfection efficiency. The LPD complex, however, is still unstable in serum due to the positive charge on the surface of the carrier.

To avoid serum instability and toxicity caused by cationic carriers, T.M. Allen and S.C. Semple used a neutral liposome to encapsulate DNA. The neutral liposome might reduce non-specific reaction between the carriers and proteins to enhance serum resistance. In addition, the neutral liposome might also prolong the half-life of DNA up to about 8∼24 hours (the original half-life of DNA is less than about 30min) and also can deliver DNA to tumor sites. The carrier, however, has difficulties entering cells due to lack of the interactions between cationic carrier and the cell, resulting in low transfection efficiency. To solve the problem, target ligand is provided to trigger endocytosis by binding with a specific receptor on the cell surface, to increase DNA transfection efficiency.

Currently, most of the clinical trials by using lipid carriers are still remained in phase II. The progress is hindered by the foregoing problems such as serum instability or toxicity of the carriers. Thus, a lipid based carrier with high serum resistance, high encapsulation efficiency, and high transfection efficiency is desirable.

WO 9851278 discloses high efficiency encapsulation of nucleic acids in lipid vesicles.

### SUMMARY

The invention provides a lipid carrier according to claim 1, comprising a lipid based particle comprising a cationic lipid, a cholesterol, a neutral phospholipid, and a neutral lipid, distearoylphosphatidylethandamine-polyethylene glycol (DSPE-PEG) wherein the cationic lipid is 100 parts by weight, the cholesterol is 50 parts by weight, the neutral phospholipid is 50 parts by weight, and the neutral lipid is 65 parts by weight.

The invention also provides a method according to claim 10 of preparing a lipid carrier, comprising the following steps. A cationic lipid, a cholesterol, a neutral phospholipid, DSPE-PEG ethanol, and water are mixed to form a lipid solution, wherein the cationic lipid is 100 parts by weight, the cholesterol is 50 parts by weight, the neutral phospholipid is 50 parts by weight, and the DSPE-PEG is 65 parts by weight. Next, a drug-containing solution is added to the lipid solution to form a suspension comprising a plurality of lipid particles, wherein the drug is encapsulated into the lipid particles. Finally, the suspension is heated to form a lipid based carrier.

A detailed description is given in the following embodiments with reference to the accompanying drawings.

### BRIEF DESCRIPTION OF THE DRAWINGS

Embodiments of the invention can be more fully understood by reading the subsequent detailed description and examples with references made to the accompanying drawings, wherein:
FIG. 1 shows drug release rates of a lipid carrier in various pH conditions of the invention.
FIG. 2 shows a comparison between transfection efficiency of the lipid carriers in serum or without serum.

### DETAILED DESCRIPTION

The invention provides a lipid carrier according to claim 1, comprising a lipid based particle comprising a cationic lipid, a cholesterol, a neutral phospholipid, and DSPE-PEG, wherein the cationic lipid is about 100 parts by weight, the cholesterol is 50 parts by weight, the neutral phospholipid is 50 parts by weight, and DSPE-PEG is 65 parts by weight.

The cationic lipid comprises 1,2-dioleoyloxy-3-(trimethylamino)propane (DOTAP), N-[1-(2,3-ditetradecyloxy)propyl]-N,N-dimethyl-N-hydroxyethylammonium bromide (DMRIE), N-[1-(2,3-dioleyloxy)propyl]-N,N-dimethyl-N-hydroxyethylammonium bromide (DORIE), N-[1-(2,3-dioleyloxy)propyl]-N,N,N-trimethylammonium chloride (DOTMA), 3β- [N- (N',N'-dimethylaminoethane)carbamyl]cholesterol (DC-Chol), or dimethyldioctadecylammonium (DDAB), preferably DOTAP.

The neutral phospholipid comprises phosphatidyl choline (PC) or phosphatidyl ethanolamine (PE), wherein the phosphatidyl choline may comprise hydrogenated soy phosphatidyl choline (HSPC). The neutral lipid is distearoylphosphatidylethanolamine-polyethyleneglycol (DSPE-PEG).

The composition of the lipid based particle is cationic lipid of 100 parts by weight, the cholesterol 50 parts by weight, the neutral phospholipid 50 parts by weight, and DSPE-PEG 65 parts by weight.

The lipid based particle is used as a drug transfection carrier when a drug is encapsulated therein, wherein the drug comprises nucleic acid drugs, proteins, peptides, or synthetic drugs, and the nucleic acid drugs further comprise plasmid DNA, antisense oligonucleotide, and RNAi. In the carrier composition, the drug and the cationic lipid have a weight ratio of 1:4~1:12, preferably 1:6.

The lipid based particle has a particle size of about 35∼95nm, a zeta potential of about -10~10mV, an encapsulation efficiency of about 85~100%, a drug release rate of about 60~70% at pH4~5, and the remained activity in serum is about 60∼100%.

Additionally, a ligand can be conjugated onto the surface of lipid based particle to recognize the receptor in a target cell. The lipid carrier with the ligand has a transfection efficiency exceeding 10 times that of a lipid carrier without a ligand.

The invention also provides a method according to claim 10, of preparing the lipid carrier. First, a cationic lipid, a cholesterol, a neutral phospholipid, a neutral lipid, ethanol, and water are mixed to form a lipid solution, wherein the cationic lipid is 100 parts by weight, the cholesterol is 50 parts by weight, the neutral phospholipid is 50 parts by weight, and the DSPE-PEG is 65 parts by weight. Next, a drug-containing solution is added to the lipid solution to form a suspension comprising a plurality of lipid based particles, wherein the drug is encapsulated into the lipid based particles. Finally, the suspension is heated to form a lipid carrier.

The solution is heated to about 50~70°C, preferably 65°C. The ethanol and water have a volume ratio of about 3:7~5:5, preferably 4:6.

The invention provides lipid based particles with uniform particle size, high encapsulation efficiency, and high serum resistance by using the specific ratio of carrier composition, ethanol/water ratios, drug/cationic lipid ratios, and heating conditions. After the lipid based particle entering the cell, the carrier will release the drug in a weak acidic lysosome in pH4∼5 due to its pH sensitivity, without decomposing by lysosome enzymes, could significantly increasing transfection efficiency of genes.

### Reference Examples

### Preparation of lipid carrier

First, 0.05mL ethanol was added to a 1.5mL centrifuge tube. Next, 0.1mg cationic DOTAP, 0.05mg cholesterol, 0.05mg HSPC, and 0.065mg DSPE-PEG were dissolved in the ethanol with water bath at 65°C. Deionized water was then added to the solution until the volume of 0.095mL and mixed completely to form a lipid solution.

Next, 0.005mL oligonucleotide-containing solution (10mg/mL) was added to the lipid solution to form a mixture comprising a plurality of lipid based particles, wherein oligonucleotide was encapsulated into the lipid based particles. Finally, 0.1mL PBS solution (pH7.4) was added to the centrifuge tube with a water bath at 65°C for 10min to form lipid carriers.

### Measurement of size and zeta potential of lipid based particle

The size of the lipid based particle was measured by Coulter N4 Plus Submicron Particle Sizer (Miami, FL) and the zeta potential thereof was measured by ZetaPlus Zeta Potential Analyzer (Brookhaven Instruments Corporation, Holtsville, NY). The measured diameter was 72.0 ± 22.5nm and the zeta potential was -0.302mV.

### Measurement of encapsulation efficiency

Encapsulated oligonucleotides and free oligonucleotides were separated by a chromatography column. The encapsulation efficiency was then measured. The calculation is illustrated in the following.

Encapsulation efficiency (%)=(total amount of oligonucleotide -the amount of free oligonucleotide)/the amount of total oligonucleotide x100%

The encapsulation efficiency of the carrier was calculated as 95 ± 5%

### Measurement of drug release rate

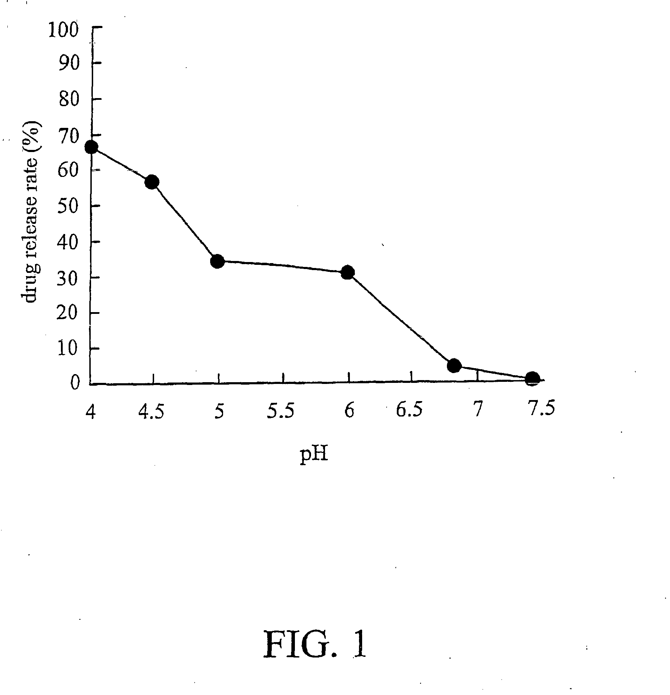

0.2mL carrier solution was mixed with 100mM buffer solutions (pH6.8, pH6, pH5.5, pH5, pH4.5, pH4), respectively, with water bath at 37°C for 5 min. After the lipid solution was separated by a Sepharose CL 4B chromatography column, the release rate of oligonucleotide was measured. Referring to FIG. 1, the carrier has a drug release rate of about 60~70% at pH4~5.

### Measurement of serum resistance

PC14PE6 cells were cultured on a 1cm² glass slide. The slide contained 2 × 10⁴ cells after culturing. Serum resistance testing was performed as the following steps. First, the cells and carriers encapsulated oligonucleotide were cultured in two mediums at 37°C for 4 hours, respectively, wherein one medium contained 10% serum and another one without serum. The cells were washed by PBS solution three times and were dissolved by mixing with another portion of PBS solution containing 1% Triton X-100 for 1 hour. Finally, the exuded cytosol was analyzed by a fluorescent analyzer (Ex: 494nm, Em: 519nm). According to the analytical results, the carrier maintained its activity within 60~100% in serum.

### Measurement of transfection efficiency of carrier with ligand

PC14PE6 cells were cultured in a 24 wells night over. Each well contained 5 × 10⁴ cells after culturing. Transfection efficiency was tested as follows. Cells and carriers with or without tamoxifen ligands were cultured in two mediums at 37°C for 4 hours, respectively, wherein one medium contained 10% serum and another one contained no serum. Next, the cells were washed by PBS solution and dissolved by mixing with another portion of PBS solution containing 1% Triton X-100 for 1 hour. Finally, the exuded cytosol was analyzed by a fluorescent analyzer (Ex: 494nm, Em: 519nm).

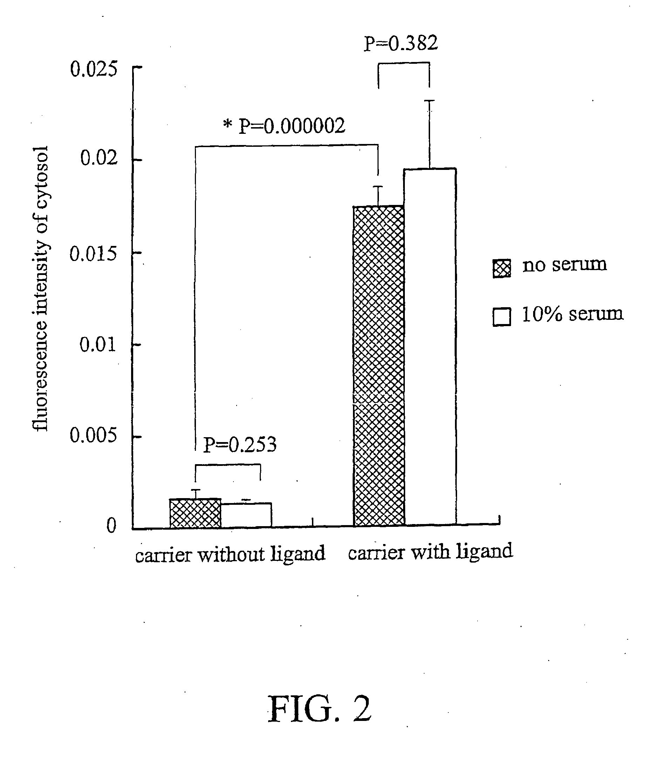

Referring to FIG. 2, the results indicate that the carrier with a ligand has a transfection efficiency exceeding 10 times that of the carrier without a ligand. Additionally, serum resistance of the lipid carrier provided by the invention has been proven because the test results under serum or without serum are the same.

While the invention has been described by way of example and in terms of preferred embodiment, it is to be understood that the invention is not limited thereto.

## Claims

1. A lipid carrier, comprising:
a lipid based particle comprising a cationic lipid, a cholesterol, a neutral phospholipid, and a distearoylphosphatidylethanolamine-polyethyleneglycol (DSPE-PEG), wherein the cationic lipid is 100 parts by weight, the cholesterol is 50 parts by weight, the neutral phospholipid is 50 parts by weight, and the DSPE-PEG is 65 parts by weight; and
a drug encapsulated into the lipid based particle, wherein the drug and the cationic lipid have a weight ratio of 1:4-1:12.

2. The lipid carrier as claimed in claim 1, wherein the cationic lipid comprises 1,2-dioleoyloxy-3-(trimethylamino)propane (DOTAP), N-[1-(2,3-ditetradecyloxy)propyl]-N,N-dimethyl-N-hydroxyethylammonium bromide (DMRIE), N-[1-(2,3-dioleyloxy)propyl]-N,N-dimethyl-N-hydroxyethylammonium bromide (DORIE), N-[1-(2,3-dioleyloxy)propyl]-N,N,N-trimethylammonium chloride (DOTMA), 3β-[N-(N',N'-dimethylaminoethane)carbamyl]cholesterol (DC-Chol), or dimethyldioctadecylammonium (DDAB).

3. The lipid carrier as claimed in claim 1, wherein the neutral phospholipid comprises phosphatidyl choline (PC) or phosphatidyl ethanolamine (PE).

4. The lipid carrier as claimed in claim 3, wherein the phosphatidyl choline comprises hydrogenated soy phosphatidyl choline (HSPC).

5. The lipid carrier as claimed in claim 1, wherein the drug comprises nucleic acid drugs, protein drugs, peptide drugs, or synthetic drugs.

6. The lipid carrier as claimed in claim 5, wherein the nucleic acid drugs comprise plasmid DNA, antisense oligonucleotide, or RNAi.

7. The lipid carrier as claimed in claim 1, wherein the drug and the cationic lipid have a weight ratio of 1:6.

8. The lipid carrier as claimed in claim 1, wherein the lipid based particle has a diameter of 35-95nm.

9. The lipid carrier as claimed in claim 1, further comprising a ligand grafted onto the lipid based particle surface that recognizes a target cell of a subject.

10. A method of preparing a lipid carrier, comprising:
mixing a cationic lipid, a cholesterol, a neutral phospholipid, a distearoylphosphatidylethanolamine-polyethyleneglycol (DSPE-PEG), ethanol, and water to form a lipid solution, wherein the cationic lipid is 100 parts by weight, the cholesterol is 50 parts by weight, the neutral phospholipid is 50 parts by weight, and the DSPE-PEG is 65 parts by weight;
adding a drug-containing solution to the lipid solution to form a solution comprising a plurality of lipid based particle, wherein the drug is encapsulated into the lipid based particle, wherein the drug and the cationic lipid have a weight ratio of 1:4-1:12; and
heating the solution to form a lipid carrier.

11. The method as claimed in claim 10, wherein the cationic lipid comprises 1,2-dioleoyloxy-3-(trimethylamino)propane (DOTAP), N-[1-(2,3-ditetradecyloxy)propyl]-N,N-dimethyl-N-hydroxyethylammonium bromide (DMRIE), N-[1-(2,3-dioleyloxy)propyl]-N,N-dimethyl-N-hydroxyethylammonium bromide (DORIE), N-[1-(2,3-dioleyloxy)propyl]-N,N,N-trimethylammonium chloride (DOTMA), 3β-[N-(N',N'-dimethylaminoethane)carbamyl]cholesterol (DC-Chol), or dimethyldioctadecylammonium (DDAB).

12. The method as claimed in claim 10, wherein the neutral phospholipid comprises phosphatidyl choline (PC) or phosphatidyl ethanolamine (PE).

13. The method as claimed in claim 12, wherein the phosphatidyl choline comprises hydrogenated soy phosphatidyl choline (HSPC).

14. The method as claimed in claim 10, wherein the ethanol and water have a volume ratio of 3:7-5:5.

15. The method as claimed in claim 10, wherein the ethanol and water have a volume ratio of 4:6.

16. The method as claimed in claim 10, wherein the drug comprises nucleic acid drugs, protein drugs, peptide drugs, or synthetic drugs.

17. The method as claimed in claim 16, wherein the nucleic acid drugs comprise plasmid DNA, antisense oligonucleotide, or RNAi.

18. The method as claimed in claim 10, wherein the drug and the cationic lipid have a weight ratio of 1:6.

19. The method as claimed in claim 10, wherein the solution is heated to 50-70°C.

20. The method as claimed in claim 10, wherein the solution is heated to 65°C.

21. The method as claimed in claim 10, wherein the lipid based particle has a diameter of 35-95nm.

22. The method as claimed in claim 10, further comprising a ligand grafted onto the lipid based particle surface that recognizes a target cell of a subject.

## Patentansprüche

1. Lipidcarrier, umfassend:
ein Teilchen auf Lipidbasis, umfassend ein kationisches Lipid, ein Cholesterol, ein neutrales Phospholipid und ein Distearoylphosphatidylethanolamin-Polyethylenglycol (DSPE-PEG), worin das kationische Lipid 100 Gewichtsteile beträgt, das Cholesterol 50 Gewichtsteile beträgt, das neutrale Phospholipid 50 Gewichtsteile beträgt und das DSPE-PEG 65 Gewichtsteile beträgt; und
einen Arzneistoff, der in das Teilchen auf Lipidbasis eingekapselt ist, worin der Arzneistoff und das kationische Lipid ein Gewichtsverhältnis von 1:4-1:12 haben.

2. Lipidcarrier nach Anspruch 1, worin das kationische Lipid 1,2-Dioleoyloxy-3-(trimethylamino)propan (DOTAP), N-[1-(2,3-Ditetradecyloxy)propyl]-N,N-dimethyl-N-hydroxyethylammoniumbromid (DMRIE), N-[1-(2,3-Dioleyloxy)propyl]-N,N-dimethyl-N-hydroxyethylammoniumbromid (DORIE), N-[1-(2,3-Dioleyloxy)propyl]-N,N,N-trimethylammoniumchlorid (DOTMA), 3β-[N-(N',N'-Dimethylaminoethan)carbamyl]cholesterol (DC-Chol) oder Dimethyldioctadecylammonium (DDAB) umfasst.

3. Lipidcarrier nach Anspruch 1, worin das neutrale Phospholipid Phosphatidylcholin (PC) oder Phosphatidylethanolamin (PE) umfasst.

4. Lipidcarrier nach Anspruch 3, worin das Phosphatidylcholin hydriertes Sojaphosphatidylcholin (HSPC) umfasst.

5. Lipidcarrier nach Anspruch 1, worin der Arzneistoff Nukleinsäurearzneistoffe, Proteinarzneistoffe, Peptidarzneistoffe oder synthetische Arzneistoffe umfasst.

6. Lipidcarrier nach Anspruch 5, worin die Nukleinsäurearzneistoffe Plasmid-DNA, Antisense-Oligonukleotid oder RNAi umfasen.

7. Lipidcarrier nach Anspruch 1, worin der Arzneistoff und das kationische Lipid ein Gewichtsverhältnis von 1:6 aufweisen.

8. Lipidcarrier nach Anspruch 1, worin das Teilchen auf Lipidbasis einen Durchmesser von 35-95 nm aufweist.

9. Lipidcarrier nach Anspruch 1, weiterhin umfassend einen auf die Oberfläche des Teilchens auf Lipidbasis aufgepfropften Liganden, welcher eine Targetzelle einer Person erkennt.

10. Verfahren zur Herstellung eines Lipidcarriers, umfassend:
Mischen eines kationischen Lipids, eines Cholesterols, eines neutralen Phospholipids, eines Distearoylphosphatidylethanolamin-Polyethylenglycols (DSPE-PEG), Ethanol und Wasser unter Bildung einer Lipidlösung, worin das kationische Lipid 100 Gewichtsteile beträgt, das Cholesterol 50 Gewichtsteile beträgt, das neutrale Phospholipid 50 Gewichtsteile beträgt und das DSPE-PEG 65 Gewichtsteile beträgt;
Zugabe einer Arzneistoff-enthaltenden Lösung zu der Lipidlösung unter Bildung einer Lösung, welche eine Vielzahl von Teilchen auf Lipidbasis umfasst, worin der Arzneistoff in das Teilchen auf Lipidbasis eingekapselt ist, worin der Arzneistoff und das kationische Lipid ein Gewichtsverhältnis von 1:4-1:12 aufweisen; und
Erwärmung der Lösung unter Bildung eines Lipidcarriers.

11. Verfahren nach Anspruch 10, worin das kationische Lipid 1,2-Dioleoyloxy-3-(trimethylamino)propan (DOTAP), N-[1-(2,3-Ditetradecyloxy)propyl]-N,N-dimethyl-N-hydroxyethylammoniumbromid (DMRIE), N-[1-(2,3-Dioleyloxy)propyl]-N,N-dimethyl-N-hydroxyethylammoniumbromid (DORIE), N-[1-(2,3-Dioleyloxy)propyl]-N,N,N-trimethylammoniumchlorid (DOTMA), 3β-[N-(N',N'-Dimethylaminoethan)carbamyl]cholesterol (DC-Chol) oder Dimethyldioctadecylammonium (DDAB) umfasst.

12. Verfahren nach Anspruch 10, worin das neutrale Phospholipid Phosphatidylcholin (PC) oder Phosphatidylethanolamin (PE) umfasst.

13. Verfahren nach Anspruch 12, worin das Phosphatidylcholin hydriertes Sojaphosphatidylcholin (HSPC) umfasst.

14. Verfahren nach Anspruch 10, worin Ethanol und Wasser ein Volumenverhältnis von 3:7-5:5 aufweisen.

15. Verfahren nach Anspruch 10, worin Ethanol und Wasser ein Volumenverhältnis von 4:6 aufweisen.

16. Verfahren nach Anspruch 10, worin der Arzneistoff Nukleinsäurearzneistoffe, Proteinarzneistoffe, Peptidarzneistoffe oder synthetische Arzneistoffe umfasst.

17. Verfahren nach Anspruch 16, worin die Nukleinsäurearzneistoffe Plasmid-DNA, Antisense-Oligonukleotid oder RNAi umfassen.

18. Verfahren nach Anspruch 10, worin der Arzneistoff und das kationische Lipid ein Gewichtsverhältnis von 1:6 aufweisen.

19. Verfahren nach Anspruch 10, worin die Lösung auf 50-70°C erwärmt wird.

20. Verfahren nach Anspruch 10, worin die Lösung auf 65°C erwärmt wird.

21. Verfahren nach Anspruch 10, worin das Teilchen auf Lipidbasis einen Durchmesser von 35-95 nm aufweist.

22. Verfahren nach Anspruch 10, weiterhin umfassend einen auf die Oberfläche des Teilchens auf Lipidbasis aufgepfropften Liganden, der eine Targetzelle einer Person erkennt.

## Revendications

1. Vecteur lipidique, comprenant:
une particule à base de lipide comprenant un lipide cationique, un cholestérol, un phospholipide neutre et un distearoylphosphatidyléthanolamine-polyéthylèneglycol (DSPE-PEG), dans lequel le lipide cationique constitue 100 parties en poids, le cholestérol constitue 50 parties en poids, le phospholipide neutre constitue 50 parties en poids et le DSPE-PEG constitue 65 parties en poids; et
un médicament encapsulé dans la particule à base de lipide, dans lequel le médicament et le lipide cationique ont un rapport de poids de 1:4 à 1:12.

2. Vecteur lipidique selon la revendication 1, dans lequel le lipide cationique comprend 1,2-dioléoyloxy-3-(triméthylamino)propane (DOTAP), N-[1-(2,3-ditétradécyloxy)propyl]-N,N-diméthyl-N-hydroxyéthylammonium bromure (DMRIE), N-[1-(2,3-dioléyloxy)propyl]-N,N-diméthyl-N-hydroxyéthylammonium bromure (DORIE), N-[1-(2,3-dioléyloxy)propyl]-N,N,N-triméthylammonium chlorure (DOTMA), 3β-[N-(N',N'-diméthylaminoéthane)carbamyl]cholestérol (DC-Chol), ou diméthyldioctadécylammonium (DDAB).

3. Vecteur lipidique selon la revendication 1, dans lequel le phospholipide neutre comprend de la choline phosphatidyl (PC) ou de l'éthanolamine phosphatidyl (PE).

4. Vecteur lipidique selon la revendication 3, dans lequel la choline phosphatidyl comprend de la choline phosphatidyl de soja hydrogénée (HSPC).

5. Vecteur lipidique selon la revendication 1, dans lequel le médicament comprend des médicaments d'acide nucléique, des médicaments de protéines, des médicaments de peptides ou des médicaments synthétiques.

6. Vecteur lipidique selon la revendication 5, dans lequel les médicaments d'acide nucléique comprennent de l'ADN de plasmide, un oligonucléotide antisens ou de l'ARNi.

7. Vecteur lipidique selon la revendication 1, dans lequel le médicament et le lipide cationique ont un rapport de poids de 1:6.

8. Vecteur lipidique selon la revendication 1, dans lequel la particule à base de lipide a un diamètre de 35 à 95 nm.

9. Vecteur lipidique selon la revendication 1, comprenant en outre un ligand greffé sur la surface de particule à base de lipide qui reconnaît une cellule cible d'un sujet.

10. Procédé de préparation d'un vecteur lipidique, comprenant:
le mélange d'un lipide cationique, un cholestérol, un phospholipide neutre, un distearoylphosphatidyléthanolamine-polyéthylèneglycol (DSPE-PEG), un éthanol et de l'eau pour constituer une solution lipidique, dans lequel le lipide cationique constitue 100 parties en poids, le cholestérol constitue 50 parties en poids, le phospholipide neutre constitue 50 parties en poids et le DSPE-PEG constitue 65 parties en poids; et
l'ajout d'une solution contenant un médicament à la solution lipidique pour constituer une solution comprenant une pluralité de particules à base de lipide, dans lequel le médicament est encapsulé dans la particule à base de lipide, dans lequel le médicament et le lipide cationique ont un rapport de poids de 1:4 à 1:12; et
le chauffage de la solution pour constituer un vecteur lipidique.

11. Procédé selon la revendication 10, dans lequel le lipide cationique comprend 1,2-dioléoyloxy-3-(triméthylamino)propane (DOTAP), N-[1-(2,3-ditétradécyloxy)propyl]-N,N-diméthyl-N-hydroxyéthylammonium bromure (DMRIE), N-[1-(2,3-dioléyloxy)propyl]-N,N-diméthyl-N-hydroxyéthylammonium bromure (DORIE), N-[1-(2,3-dioléyloxy)propyl]-N,N,N-triméthylammonium chlorure (DOTMA), 3β-[N-(N',N'-diméthylaminoéthane)carbamyl]cholestérol (DC-Chol), ou diméthyldioctadécylammonium (DDAB).

12. Procédé selon la revendication 10, dans lequel le phospholipide neutre comprend de la choline phosphatidyl (PC) ou de l'éthanolamine phosphatidyl (PE).

13. Procédé selon la revendication 12, dans lequel la choline phosphatidyl comprend de la choline phosphatidyl de soja hydrogénée (HSPC).

14. Procédé selon la revendication 10, dans lequel l'éthanol et l'eau ont un rapport de volume de 3:7 à 5:5.

15. Procédé selon la revendication 10, dans lequel l'éthanol et l'eau ont un rapport de volume de 4:6.

16. Procédé selon la revendication 10, dans lequel le médicament comprend des médicaments d'acide nucléique, des médicaments de protéines, des médicaments de peptides ou des médicaments synthétiques.

17. Procédé selon la revendication 16, dans lequel les médicaments d'acide nucléique comprennent de l'ADN de plasmide, un oligonucléotide antisens ou de l'ARNi.

18. Procédé selon la revendication 10, dans lequel le médicament et le lipide cationique ont un rapport de poids de 1:6.

19. Procédé selon la revendication 10, dans lequel la solution est chauffée de 50 à 70°C.

20. Procédé selon la revendication 10, dans lequel la solution est chauffée à 65°C.

21. Procédé selon la revendication 10, dans lequel la particule à base de lipide a un diamètre de 35 à 95 nm.

22. Procédé selon la revendication 10, comprenant en outre un ligand greffé sur la surface de particule à base de lipide qui reconnaît une cellule cible d'un sujet.
